# EUROPEAN PATENT APPLICATION

(11) **EP 2 233 143 A1**
(43) Date of publication of application: **29.09.2010**
(21) Application number: 09290216.2
(22) Date of filing: 24.03.2009
(51) Int. Cl.: A61K 31/7056, A61P 7/02

(54) **Use of idrabiotaparinux for decreasing the incidence of bleedings during an antithrombotic treatment**

(71) Applicant: Sanofi-Aventis, 75013 Paris (FR)
(72) Inventor: Cariou, Roger, 75013 Paris (FR); Chew, Paul, 08807-0800 Bridgewater (US); Destors, Jean-Michel, 75013 Paris (FR); Pillion, Gérard, 75013 Paris (FR); Silvestre, Louise, 75013 Paris (FR)
(74) Representative: Romanowski, Caroline

(57) **Abstract**

The invention relates to the use of idrabiotaparinux for the treatment and secondary prevention of thrombotic pathologies, wherein the use of idrabiotaparinux involves a decrease in the incidence of bleedings, in particular major bleedings, during said treatment.

## Description

The invention relates to the use of idrabiotaparinux for preventing the incidence of bleedings during an antithrombotic treatment.

The standard treatment for venous thromboembolism (hereafter "VTE"), i.e. both deep venous thrombosis (hereafter "DVT") and pulmonary embolism (hereafter "PE"), is an initial course of unfractionated heparin or low-molecular weight heparin for at least 5 days, overlapping with a vitamin K antagonist (hereafter "VKA") for at least 4 to 5 days (until a stable, therapeutic value of international normalized ratio (hereafter "INR") is reached), and followed by VKA alone for 3 or 6 months or for a longer duration depending on the risk factors for subsequent VTE recurrences (H.R. Büller et al., Chest, 2004, 126, suppl. 3, 401S-428S; D.J. Quinlan et al., Ann. Intern. Med., 2004, 140, 175-83).

This therapy is effective, but the extended use of VKA for prophylaxis of VTE recurrences is often constrained by risk-benefit limitations and inconvenience. Indeed, because of numerous food and drug interactions and a narrow therapeutic margin, their use requires regular laboratory monitoring and dose-adjustments to reduce the risk of thrombotic recurrence (underanticoagulation) or bleeding (overanticoagulation) (J. Ansell et al., Chest, 2008,133, 160S-198S). For these reasons, VKA are often discontinued before the recommended period of 3 to 12 months of prophylaxis for idiopathic thromboembolism (N. Engl. J. Med., 2007, 357, 11, 1105-1112).

Idrabiotaparinux, developed by sanofi-aventis, is the biotinylated pentasaccharide corresponding to the structure depicted below. The pentasaccharide structure of idrabiotaparinux is the same as idraparinux, another antithrombotic agent developed by sanofi-aventis (see structure below). However in idrabiotaparinux, the presence of a biotin hook covalently linked to the first saccharidic unit enables the compound to be neutralized by avidin or streptavidin, as described in the international patent application WO 02/24754.

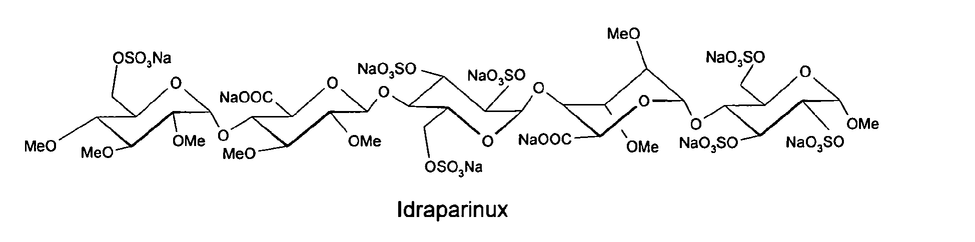

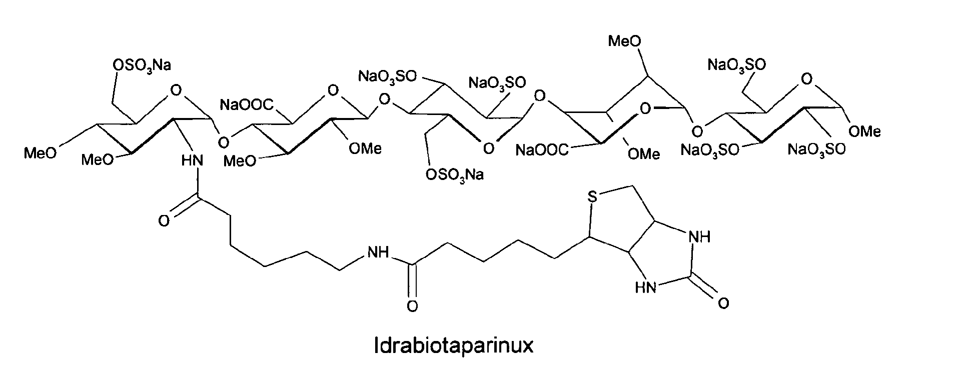

In the EQUINOX trial, which enrolled patients with DVT treated for 6 months with equimolar doses of either idrabiotaparinux or idraparinux, idrabiotaparinux, with the same anti-activated factor X pharmacological activity (hereafter "anti-Xa activity") as idraparinux, was shown to have a similar efficacy, but, surprisingly, a better safety with less observed bleedings, in particular major bleedings.

Therefore, the subject-matter of the invention is the use of idrabiotaparinux for the manufacture of a medicament useful for the treatment and secondary prevention of thrombotic pathologies, wherein the use of idrabiotaparinux involves a decrease in the incidence of bleedings during said treatment.

In other words, the invention relates to the use of idrabiotaparinux as an antithrombotic treatment, wherein said use minimizes the risk of bleedings during the antithrombotic treatment. Indeed, idrabiotaparinux enables to increase the benefit-risk ratio during the antithrombotic treatment.

According to the instant invention, the term "bleedings" designates any clinically relevant bleeding (major or clinically relevant non-major hemorrhage).

According to the instant invention, the term "major bleedings" designates the following clinical situations:
- bleeding associated with a fall in hemoglobin of 2 g per deciliter or more,
- bleeding that led to a transfusion of 2 or more units of packed red cells or whole blood (a red cell unit being defined as the quantity of red cells obtained from or corresponding to approximately 500 ml of whole blood),
- bleeding that involved a critical organ (intracranial, intraocular, intraspinal, retroperitoneal or pericardial), and
- bleeding that contributed to death;
   whereas the term "clinically relevant non-major bleeding" designates the following clinical situations:

- any bleeding compromising hemodynamics,
- any bleeding leading to hospitalization,
- subcutaneous hematoma larger than 25 cm², or 100 cm² if there was a traumatic cause,
- intramuscular hematoma documented by ultrasonography,
- epistaxis that lasted for more than 5 minutes, was repetitive (i.e., two or more episodes of bleeding more extensive than spots on a handkerchief within 24 hours), or led to an intervention (e.g., packing or electrocoagulation),
- gingival bleeding occurring spontaneously (i.e., unrelated to eating or tooth brushing) or lasting for more than 5 minutes,
- hematuria that was macroscopic and was spontaneous or lasted for more than 24 hours after instrumentation (e.g., catheter placement or surgery) of the urogenital tract,
- macroscopic gastrointestinal hemorrhage, including at least one episode of melena or hematemesis, if clinically apparent with positive results on a fecal occult-blood test,
- rectal blood loss, if more than a few spots on toilet paper,
- hemoptysis, if more than a few speckles in the sputum and not occurring within the context of pulmonary embolism, and
- any other bleeding type considered to have clinical consequences for a patient:
   - such as medical intervention, the need for unscheduled contact (visit or telephone call) with a physician, or temporary cessation of a study drug,
   - or associated with pain or impairment of activities of daily life.

According to the instant invention, the decrease in the incidence of bleedings, or the effect of minimizing the risk of bleedings, are meant in comparison with the incidence rate and risk, respectively, of either a treatment with idraparinux or of a standard antithrombotic treatment.

The term "treatment" as used herein refers to the administration of a therapy to an individual who already manifests at least one symptom of a disease or condition (in the instant case, a thrombotic pathology such as confirmed venous thromboembolism, in particular deep venous thrombosis) or who has previously manifested at least one symptom of such a disease or condition. The term "treatment" in the framework of the instant invention therefore encompasses both a curative treatment (i.e., treatment of a confirmed thrombotic pathology) and the secondary prevention of thrombotic pathologies (i.e., prevention of recurrences of thrombotic events).

In an embodiment of the invention, idrabiotaparinux is administered for up to 6 months.

In another embodiment of the invention, the incidence of bleedings is decreased compared with a treatment by either idraparinux or a vitamin K antagonist, such as warfarin or acenocoumarol.

In another embodiment of the invention, idrabiotaparinux is used for the manufacture of a medicament useful for the treatment (curative treatment) of venous thromboembolism, such as deep venous thrombosis, and for the prevention of subsequent venous thromboembolic events (i.e., prevention of venous thromboembolic events recurrences).

In another embodiment of the invention, the decrease in the incidence of bleedings is assessed after 6 months of treatment with idrabiotaparinux.

The use according to the instant invention is more particularly directed to decreasing the incidence of major bleedings, as defined above.

The invention is also directed to the use of idrabiotaparinux for the manufacture of a medicament useful for the treatment and secondary prevention of thrombotic pathologies, wherein the use of idrabiotaparinux involves an increase in the benefit-risk ratio during said treatment.

The invention is also directed to the use of idrabiotaparinux for the manufacture of a medicament useful for the treatment and secondary prevention of thrombotic pathologies, wherein the use of idrabiotaparinux involves an improved safety during said treatment.

In another embodiment, the invention relates to a method for decreasing the incidence of bleedings, in particular major bleedings, during an antithrombotic treatment, wherein the drug administered is idrabiotaparinux. More particularly, the invention relates to a method for the treatment and secondary prevention of thrombotic pathologies, wherein the drug administered is idrabiotaparinux and wherein the method involves a decrease in the incidence of bleedings during said treatment.

The method according to the invention advantageously comprises the step of administering to a patient in need thereof a treatment with idrabiotaparinux.

In said method, the treatment with idrabiotaparinux is advantageously administered for up to 6 months. The treatment with idrabiotaparinux is advantageously administered at a dose of 3.0 mg once-weekly, preferably by the subcutaneous route.

In another embodiment, the invention relates to a pharmaceutical composition comprising idrabiotaparinux, useful for decreasing the incidence of bleedings, in particular major bleedings, during an antithrombotic treatment. Such a pharmaceutical composition advantageously comprises idrabiotaparinux, at a weekly subcutaneous dose of 3.0 mg for example, as well as pharmaceutically acceptable and inert excipients. Such excipients are chosen among those known in the Art, according to the desired pharmaceutical formulation and mode of administration. An advantageous pharmaceutical composition according to the invention is an injectable formulation adapted to the subcutaneous route.

The invention will be more clearly understood by reference to the following examples of the invention, which are included herewith for purposes of illustration only and are not intended to be limiting the invention.

### 1) EQUINOX clinical trial

### - Objectives and trial design

This trial was aimed at studying the bioequipotency of idrabiotaparinux and idraparinux, as assessed by anti-Xa activity at month 6, in patients with acute symptomatic DVT, as well as studying the ability to neutralize idrabiotaparinux with avidin, and also at documenting the safety and efficacy of idrabiotaparinux and idraparinux.

The study was an international, multi-center, double-blind, randomized, with two parallel groups phase III study, in patients treated for acute symptomatic DVT. In order to get 600 patients (300 patients in each treatment group) who completed the 6-month treatment with idrabiotaparinux or idraparinux, 700 patients with confirmed symptomatic DVT of the lower limbs were to be randomized to either idrabiotaparinux (n = 350 patients) or idraparinux (n = 350 patients). 300 completed patients per group, with a 6-month extent of exposure to the drug, were considered as an adequate sample size to assess clinical safety of idrabiotaparinux, i.e. to characterize the pattern of adverse events over time.

During the study period, patients were treated with equimolar doses of idrabiotaparinux (3.0 mg) or of idraparinux (2.5 mg). The drug was injected subcutaneously, once-weekly.

For safety, the main endpoints were clinically relevant bleeding (major or clinically relevant non-major), as classified by the Central Independent Adjudication Committee (hereafter "CIAC"), and death (cause of death validated by the CIAC).

The safety population consisted of all randomized patients who took at least one dose of study medication (all treated population). Patients were analyzed according to the treatment they actually received.

The main safety analysis period was the randomized treatment period, defined as the period from first study drug administration (taken as Day 1) up to Day 182 (hereafter "D182").

The numbers and percentages of patients with clinically relevant bleeding within 6 months (from first study drug administration up to Day 182) were presented by treatment group. The rate of any clinically relevant bleeding was calculated by dividing the number of patients with this event by the number of treated patients (crude rate). Bleeding rate estimates and exact 95% confidence interval (hereafter "CI") were presented by treatment group. Cumulative incidences of any clinically relevant bleeding during the on-treatment period were also described by treatment group using the Kaplan-Meier method. Time to first clinically relevant bleeding was censored at the minimum day between: Day 182; the day of last study drug administration + 7 days; the day of death ; and the day of last clinical event assessment giving any information on bleeding symptoms.

The numbers and percentages (crude rates) of patients with major bleedings within 6 months (from first study drug administration up to Day 182) were also presented by treatment group. The criteria used by the CIAC to classify major bleedings were summarized according to the worst severity. The order of severity was: fatal bleeding, including fatal intra-cranial hemorrhage (hereafter "ICH"), non-fatal bleeding within a critical organ, non-fatal bleeding with fall in hemoglobin or transfusion.

### - Results

The all randomized population included a total of 757 patients: 386 patients were randomly assigned to receive idrabiotaparinux, and 371 to receive idraparinux. Two randomized patients, one in the idrabiotaparinux group and one in the idraparinux group, did not receive any idrabiotaparinux/idraparinux injection. All other patients received the appropriate treatment as assigned by the study protocol.

The number of patients with any bleeding (clinically relevant major or non-major bleeding, as confirmed by CIAC) and the number of patients with major bleeding up to Day 182 were lower in the idrabiotaparinux group, with respect to the idraparinux group (see Table 1). In particular as regards major bleedings, an incidence of 0.8% was observed in the idrabiotaparinux group, versus a 3.8% incidence rate in the idraparinux group (p=0.006 according to Fisher's exact test).

**Table 1 - Number (% and exact [95%CI]) of patients with bleeding from first study drug administration up to D182 - All treated population**

| | ldrabiotaparinux (N=385) | Idraparinux (N=370) |
|---|---|---|
| Any bleeding [n (%)] | 20 (5.2%) | 27 (7.3%) |
| (95% CI) | (3.2 to 7.9) | (4.9 to 10.4) |
| Major bleeding [n (%)] | 3 (0.8%) | 14 (3.8%) |
| (95% CI) | (0.2 to 2.3) | (2.1 to 6.3) |

| | | |
|---|---|---|
| CI = Confidence Interval | | |

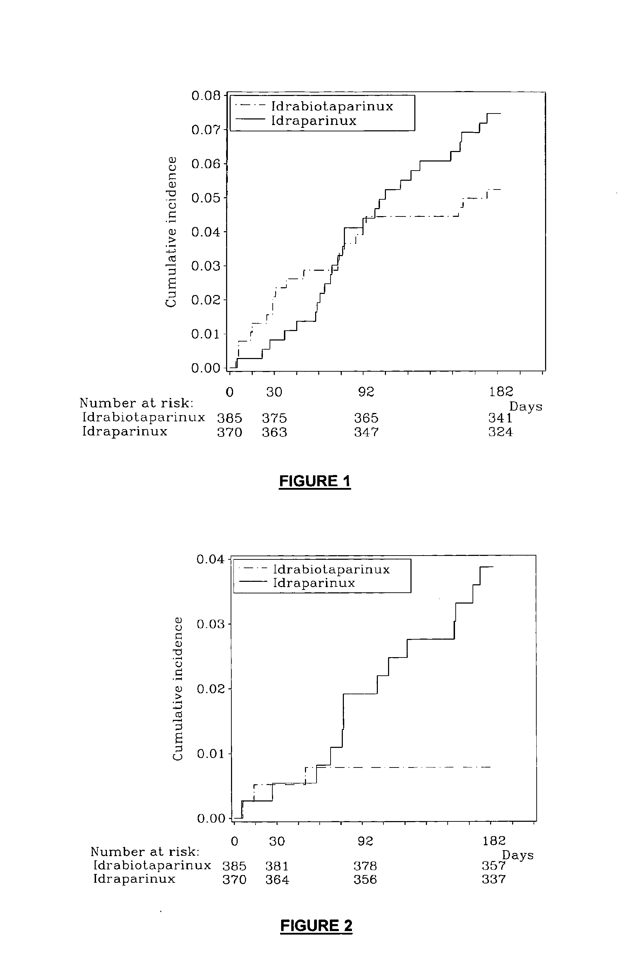

Figure 1 represents the Kaplan-Meier cumulative incidence curves of first bleeding (any bleeding) during the on-treatment period, considering all treated population. As apparent in figure 1, at month 3 the bleeding incidences in the two treatment groups were comparable. From month 3 up to month 6, the bleeding incidence in the idraparinux group continued to increase regularly, to reach a value higher than in the idrabiotaparinux group at month 6.

Figure 2 represents the Kaplan-Meier cumulative incidence curves of first major bleeding during the on-treatment period, considering all treated population. As apparent in figure 2, after month 2 there is a marked difference in major bleeding incidences between the two treatment groups, in favour of the idrabiotaparinux group.

As detailed in Table 2, five intracranial hemorrhages were observed, all in the idraparinux group; three of them were fatal. One patient in the idrabiotaparinux group also experienced a fatal bleeding, due to a suicide by cutting veins.

**Table 2 - Number (%) of patients with major bleedings from first study drug administration up to D182 by adjudication criterion - All treated population**

| | | ldrabiotaparinux | ldraparinux |
|---|---|---|---|
| | | (N=385) | (N=370) |
| Any major bleeding [n(%)] | | 3 (0.8%) | 14 (3.8%) |
| Fatal bleeding: | | | |
| | . Intracranial | 0 | 3 (0.8%) |
| | . Gastrointestinal | 0 | 0 |
| | . Other | 1 (0.3%) | 0 |
| Non-fatal bleeding into a critical organ: | | | |
| | . Intracranial | 0 | 2 (0.5%) |
| | . Retroperitoneal | 0 | 2 (0.5%) |
| | . Pericardial | 0 | 0 |
| | . Other | 0 | 0 |
| Non-fatal bleeding associated with a fall | | | |
| in hemoglobin >=2g/dL and/or leading to | | | |
| a transfusion >=2 units of blood (whole | | | |
| blood and/or packed red blood cells) | | 2 (0.5%) | 7 (1.9%) |

### 2) Other comparators

In the clinical trial VAN GOGH, idraparinux (2.5 mg once weekly by subcutaneous route) was compared to standard therapy (namely unfractionated heparin or low-molecular weight heparin followed by a VKA antagonist such as warfarin or acenocoumarol) for the prevention of recurrent venous thromboembolism.
According to the results published in N. Engl. J. Med., 2007, 357, 1094-104, the incidence of clinically relevant bleedings (any bleedings) at 6 months after start of the treatment (Day 183) in patients with DVT were 8.3% in the idraparinux group and 8.1 % in the standard therapy group. The corresponding rates for major bleedings were 1.9% and 1.5%, respectively.
For major bleedings, the putative odds ratio (OR) comparing idrabiotaparinux to the standard treatment (OR= 0.249 [95% CI: 0.061-1.020]) yielded a p value of 0.053; this putative OR was calculated as OR of idrabiotaparinux versus idraparinux in EQUINOX (0.200) times OR of idraparinux versus standard treatment in van Gogh DVT (1.245).

In the clinical trial THRIVE, ximelagatran, an oral direct thrombin inhibitor, was compared to standard enoxaparin/warfarin (VKA) treatment for the prevention of recurrent venous thromboembolism. According to the results published in the Journal of American Medical Association (JAMA), 2005, 293, no. 6, 681-689, the incidence of clinically relevant bleedings at 6 months after start of the treatment was 7.6% in the group treated with VKA, with a 2.2% rate for major bleedings.

These results suggest that the treatment with idrabiotaparinux implies a lower incidence of bleedings, in particular major bleedings, compared either to idraparinux or to standard thromboembolic therapies such as VKA.

## Claims

1. Use of idrabiotaparinux for the manufacture of a medicament useful for the treatment and secondary prevention of thrombotic pathologies, wherein the use of idrabiotaparinux involves a decrease in the incidence of bleedings during said treatment.

2. The use according to claim 1, wherein idrabiotaparinux is administered for up to 6 months.

3. The use according to claim 1 or claim 2, wherein the thrombotic pathology is venous thromboembolism.

4. The use according to any of claims 1 to 3, wherein the thrombotic pathology is deep venous thrombosis.

5. The use according to any of claims 1 to 4, wherein said medicament is useful for the treatment of venous thromboembolism and for the prevention of subsequent venous thromboembolic events.

6. The use according to any of claims 1 to 5, wherein the incidence of bleedings is decreased compared with a treatment by either idraparinux or a vitamin K antagonist.

7. The use according to any of claims 1 to 6, wherein the decrease in the incidence of bleedings is assessed after 6 months of treatment with idrabiotaparinux.

8. The use according to any of claims 1 to 7, wherein the use of idrabiotaparinux involves a decrease in the incidence of major bleedings.

9. Use of idrabiotaparinux for the manufacture of a medicament useful for the treatment and secondary prevention of thrombotic pathologies, wherein the use of idrabiotaparinux involves an increase in the benefit-risk ratio during said treatment.

10. Use of idrabiotaparinux for the manufacture of a medicament useful for the treatment and secondary prevention of thrombotic pathologies, wherein the use of idrabiotaparinux involves an improved safety during said treatment.
